# EUROPÄISCHE PATENTSCHRIFT

(11) **EP 0 474 074 B1**
(45) Veröffentlichungstag und Bekanntmachung des Hinweises auf die Patenterteilung: **08.06.1994**
(21) Anmeldenummer: 91114226.3
(22) Anmeldetag: 24.08.1991
(51) Int. Cl.: C07C 25/08, C07C 17/12

(54) **Verfahren zur Herstellung von Dichlorbenzol mit erhöhtem p-Anteil**
Process for preparing dichlorobenzene with high content of the p-isomer
Procédé pour la préparation de dichlorobenzène à teneur élevée en p-isomère

(30) Priorität: 06.09.1990 DE 4028269
(43) Veröffentlichungstag der Anmeldung: 11.03.1992
(73) Patentinhaber: BAYER AG, 51368 Leverkusen (DE)
(72) Erfinder: Mais, Franz-Josef, Dr., W-4000 Düsseldorf 1 (DE); Fiege, Helmut, Dr., W-5090 Leverkusen 1 (DE)

(56) Entgegenhaltungen:
- EP-B- 0 126 669
- CHEMICAL ABSTRACTS, Band 104, Nr. 11, 17. März 1986, Columbus, Ohio, USA; J. KIJI et al: "Phenothiazine derivatives", Seite 663, Zusammenfassung Nr. 88576s; & JP-A-60136576
- CHEMICAL ABSTRACTS, Band 108, Nr. 10, 7. März 1988, Columbus, Ohio, USA; J. KIJI et al: "Preparation and properties of selectively p-chlorinated polystyrene", Seite 10, Zusammenfassung Nr. 76 027h
- CHEMICAL ABSTRACTS, Band 104, Nr. 11, 17. März 1986, Columbus, Ohio, USA; IHARA CHEMICAL INDUSTRY: "Ring-halogenation catalyst for alkylbenzenes", Seite 630, Zusammenfassung Nr. 88 242e; & JP-A-60125251

## Beschreibung

Die vorliegende Erfindung betrifft die Herstellung von Dichlorbenzol mit erhöhtem p-Anteil durch Kernchlorierung von Benzol oder Chlorbenzol in Gegenwart von Friedel-Crafts-Katalysatoren und in Gegenwart von Co-Katalysatoren in flüssiger Phase.

p-Dichlorbenzol ist ein wertvolles Zwischenprodukt zur Synthese beispielsweise von Farbstoff- und Pharmavorprodukten; es dient weiterhin als wichtiges Monomer zur Herstellung des hochwertigen Kunststoffes Polyphenylensulfid.

Die Kernchlorierung von Benzol ergibt zunächst Chlorbenzol, das weiter zu einem Gemisch der drei isomeren Dichlorbenzole chloriert werden kann.

Daher kann zur Herstellung von Dichlorbenzol auch von Chlorbenzol ausgegangen werden.

Im allgemeinen führt man die Chlorierung in flüssiger Phase mit gasförmigem Chlor und in Gegenwart von Friedel-Crafts-Katalysatoren, wie beispielsweise Eisen-(III)-chlorid, durch.

Durch zusätzliche Co-Katalysatoren zum Friedel-Crafts-Katalysator kann die Isomeren-Zusammensetzung der Dichlorbenzolmischung so verändert werden, daß ein größerer Anteil an p-Dichlorbenzol gebildet wird. Solche Co-Katalysatoren sind beispielsweise Schwefel oder Dischwefeldichlorid (Ullmann's Encylopedia of Industrial Chemistry, 5. Ed., Vol. A6, Seite 336) oder bestimmte Phenothiazine, die chlor- oder bromhaltige Substituenten am N-Atom tragen (EP 126 669).

Weiterhin ist die Chlorierung von Benzol oder Chlorbenzol in Gegenwart von bestimmten Zeolithen bekannt (EP 118 851, EP 195 514, EP 225 723, EP 231 133, EP 273 736, US 4 777 305); auch bei dieser Variante in Gegenwart von Zeolithen können weitere co-katalytisch wirkende Substanzen zugesetzt werden (EP 154 236, EP 231 662, DE-OS 37 20 391, EP 248 931). Nachteilig an der Katalyse durch Zeolithe ist im allgemeinen der hohe Bedarf von 2 bis 6-Gew.-% an Zeolith, bezogen auf das Substrat, und weiterhin die Tatsache, daß hierbei im allgemeinen das anfallende Chlorierabgas neben HCl noch deutliche Mengen an ungenutzt entweichendem Chlor enthält.

Es wurde nun ein Verfahren zur Herstellung von Dichlorbenzol mit erhöhtem p-Anteil durch Kernchlorierung von Benzol oder Chlorbenzol in Gegenwart von Friedel-Crafts-Katalysatoren und in Gegenwart von Co-Katalysatoren in flüssiger Phase gefunden, das dadurch gekennzeichnet ist, daß man als Co-Katalysator eines oder mehrere substituierte Phenothiazine der Formel 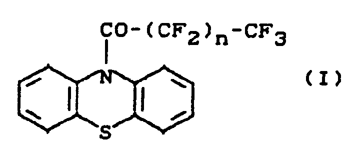
einsetzt, in der
- n: den Wert Null, Eins oder Zwei annimmt.

Als erfindungsgemäße Co-Katalysatoren kommen in Frage: N-Trifluoracetyl-phenothiazin, N-Pentafluorpropionylphenothiazin, N-Heptafluorbutyryl-phenothiazin. In bevorzugter Weise wird N-Trifluoracetyl-phenothiazin, worin also der Index n den Wert Null annimmt, als Co-Katalysator eingesetzt.

Die erfindungsgemäß einsetzbaren Co-Katalysatoren lassen sich durch grundsätzlich bekannte Methoden, beispielsweise durch Umsetzung von Phenothiazin mit perfluorierten Carbonsäureanhydriden oder perfluorierten Carbonsäurechloriden, herstellen.

Das erfindungsgemäße Verfahren wird in flüssiger Phase durchgeführt, wobei das Substrat Benzol oder Chlorbenzol oder ein Gemisch von ihnen auch mit einem gegen Chlor inerten Lösungsmittel verdünnt werden kann, In bevorzugter Weise wird ohne Lösungsmittel gearbeitet. Als Chloriermittel wird Chlor gasförmig in den Reaktionsansatz eingeleitet.

Der Reaktionsdruck ist grundsätzlich unkritisch; er kann normal, erniedrigt oder erhöht sein, In bevorzugter Weise wird bei Normaldruck gearbeitet.

Als Reaktionstemperatur für die erfindungsgemäße Kernchlorierung kommt grundsätzlich der Bereich zwischen dem Erstarrungspunkt und dem Siedepunkt des Reaktionsgemisches in Frage. In bevorzugter Weise liegt die Reaktionstemperatur bei 10-80°C, besonders bevorzugt bei 40-70°C.

Der Wassergehalt der Reaktionsmischung ist im allgemeinen unkritisch, Es ist daher bevorzugt, alle Einsatzstoffe nicht speziell zu trocknen, sondern sie mit dem (geringen) Wassergehalt einzusetzen, mit dem sie üblicherweise in der chemischen Technik vorliegen. Es ist jedoch auch möglich, alle oder einzelne Einsatzstoffe zu trocknen. Im allgemeinen sollte der Wassergehalt der Einsatzstoffe nicht über den Sättigungsgrenzen bei der gewählten Reaktionstemperatur liegen. Bevorzugt liegen die Wassergehalte im Reaktionsgemisch nicht über 250 ppm, besonders bevorzugt nicht über 100 ppm.

Als Friedel-Crafts-Katalysatoren kann man die üblichen, dem Fachmann hierfür bekannten Lewis-Säuren oder Elemente, die unter den Reaktionsbedingungen Lewis-Säuren bilden, einsetzen. Das sind beispielsweise die Elemente Eisen, Antimon, Aluminium, Gallium und weitere dem Fachmann bekannte oder deren Chalkogenide oder Halogenide, wie beispielsweise Eisen-(III)-chlorid, Antimon-(III)-chlorid, Aluminiumchlorid, Antimonoxychlorid, Eisensulfid und weitere, In bevorzugter Weise wird Eisen oder Eisen-(III)-chlorid oder ein Gemisch davon eingesetzt.

Die Mengen des erfindungsgemäß einzusetzenden Friedel-Crafts-Katalysators liegen im Bereich von 0,001-1 Gew.-%, bevorzugt im Bereich von 0,01-0,1 Gew,-%, bezogen auf Benzol oder Chlorbenzol oder deren Gemisch.

Die Mengen der erfindungsgemäß einzusetzenden Co-Katalysatoren liegen im Bereich von 0,001-2 Gew.-%, bevorzugt im Bereich von 0,01-0,2 Gew.-%, bezogen auf Benzol oder Chorbenzol oder deren Gemisch. Dabei ist die Einsatzmenge an Co-Katalysator so zu wählen, daß das Molverhältnis von Friedel-Crafts-Katalysator und Co-Katalysator in einem Bereich von 10:1-1:10 liegt. Bevorzugt wird ein Molverhältnis von 2:1-1:2, besonders bevorzugt von etwa 1:1 eingestellt.

Das erfindungsgemäße Verfahren ergibt bei der Dichlorierung von Benzol bzw. der Chlorierung von Chlorbenzol oder der Chlorierung eines Gemisches beider einen besonders hohen Anteil an p-Dichlorbenzol. Der stark paradirigierende Effekt der erfindungsgemäß einzusetzenden Co-Katalysatoren ist ausgesprochen überraschend, weil die analogen Chlor- bzw. Bromverbindungen wie sie in EP 126 669 beschrieben sind, nur einen geringen Effekt zeigen. Dies hat offenbar dazu geführt, daß die analogen Fluorverbindungen, die erfindungsgemäß eingesetzt werden, als wirkungslos angesehen wurden und in EP 126 669 ausdrücklich nicht in Betracht gezogen wurden. Gerade diese Fluorverbindungen gemäß vorliegender Erfindung zeigen eine unerwartete sprunghafte Steigerung des p-Dichlorbenzolanteils im Reaktionsprodukt.

### Beispiele

### Beispiel 1

Man legte 100 Gew.-Teile Benzol in einem Reaktor vor und gab unter Rühren 0,050 Gew.-Teile Eisen(III)-chlorid und 0,091 Gew.-Teile des Co-Katalysators der Formel (I) mit n=0 vor. Dann wurde auf 60°C erhitzt, und bei dieser Temperatur wurden 127 Gew.-Teile Cl₂ (140 Mol-% Cl₂, bezogen auf Benzol) im Verlaufe von 5 Stunden gleichmäßig eingeleitet. Die gaschromatische (GC-)Analyse des Reaktionsgemisches ergab folgende Zusammensetzung:
0,12 % Benzol
42,90 % Chlorbenzol
9,83 % ortho-Dichlorbenzol
0,08 % meta-Dichlorbenzol
46,98 % para-Dichlorbenzol
0,09 % Trichlorbenzole und höhere Chlorbenzole
Das entspricht einem Verhältnis von ortho- zu para-Dichlorbenzol von o/p = 0,21.

### Beispiel 2

Das Verfahren von Beispiel 1 wurde wiederholt, nur daß statt der dortigen Co-Katalysatormenge 0,112 Gew.-Teile des Co-Katalysators der Formel (I) mit n=0 vorgelegt wurden und daß 136,7 Gew.-Teile Cl₂ (150 Mol-% Cl₂, bezogen auf Benzol) eingeleitet wurden. Nach der Umsetzung war die GC-Zusammensetzung wie folgt:
0,02 % Benzol
30,82 % Chlorbenzol
11,70 % ortho-Dichlorbenzol
0,10 % meta-Dichlorbenzol
57,27 % para-Dichlorbenzol
0,09 % Trichlorbenzole und höhere Chlorbenzole
Das entspricht einem Verhältnis o/p = 0,20.

### Beispiel 3

Das Verfahren von Beispiel 1 wurde wiederholt, nur daß statt der dortigen Co-Katalysatormengen 0,51 Gew.-Teile Eisen(III)-chlorid und 0,93 Gew.-Teile des Co-Katalysators der Formel (I) mit n=0 eingesetzt wurden und daß bei 20°C chloriert wurde. Die Zusammensetzung des Chlorierungsgemisches war wie folgt:
0,38 % Benzol
53,79 % Chlorbenzol
6,33 % ortho-Dichlorbenzol
0,03 % meta-Dichlorbenzol
39,36 % para-Dichlorbenzol
0,11 % Trichlorbenzole und höhere Chlorbenzole
Damit ergab sich o/p = 0,16.

### Beispiel 4

Das Verfahren von Beispiel 1 wurde wiederholt, nur daß statt des dortigen Co-Katalysators 0,109 Gew.-Teile des Co-Katalysators der Formel (I) mit n=1 eingesetzt wurden. Die Zusammensetzung des Chlorierungsgemisches war wie folgt:
0,20 % Benzol
46,73 % Chlorbenzol
9,55 % ortho-Dichlorbenzol
0,07 % meta-Dichlorbenzol
43,40 % para-Dichlorbenzol
0,05 % Trichlorbenzole und höhere Chlorbenzole
Damit ergab sich ein Verhältnis von o/p = 0,22.

### Beispiel 5

Das Verfahren von Beispiel 1 wurde wiederholt, nur daß statt des dortigen Co-Katalysators 0,125 Gew.-Teile des Co-Katalysators der Formel (I) mit n=2 eingesetzt wurden. Die Zusammensetzung des Chlorierungsgemisches war wie folgt:
0,30 % Benzol
47,33 % Chlorbenzol
9,76 % ortho-Dichlorbenzol
0,10 % meta-Dichlorbenzol
42,45 % para-Dichlorbenzol
0,06 % Trichlorbenzole und höhere Chlorbenzole
Das ergab ein Verhältnis von o/p = 0,23.

### Beispiel 6

Man legte in einem Reaktor 100 Gew.-Teile Chlorbenzol, 0,06 Gew.-Teile Eisen-(III)-chlorid und 0,11 Gew.-Teile des Co-Katalysators der Formel (I) mit n=0 vor. Unter Rühren erhitzte man auf 70°C und leitete bei dieser Temperatur 37,9 Gew.-Teile Cl₂ (60 Mol-% Cl₂, bezogen auf Chlorbenzol) im Verlaufe von 2,5 Stunden gleichmäßig ein. Die GC-Analyse des Chlorierungsgemisches ergab danach folgende Zusammensetzung:
35,07 % Chlorbenzol
11,69 % ortho-Dichlorbenzol
0,09 % meta-Dichlorbenzol
53,14 % para-Dichlorbenzol
0,01 % Trichlorbenzole und höhere Chlorbenzole
Das ergab somit ein Verhältnis von o/p = 0,22.

### Vergleichsbeispiel 7

Das Verfahren von Beispiel 1 wurde wiederholt, nur daß statt des dortigen Co-Katalysator 0,106 Gew.-Teile des Co-Katalysators (aus EP 126 669) der Formel 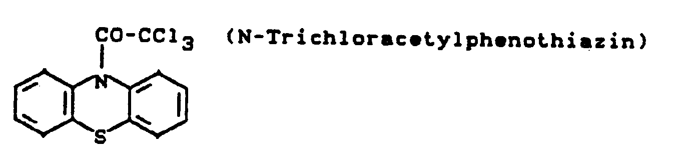
einsetzt wurden. Nach der Chlorierung war die Zusammensetzung wie folgt:
2,16 % Benzol
40,46 % Chlorbenzol
16,23 % ortho-Dichlorbenzol
0,70 % meta-Dichlorbenzol
40,08 % para-Dichlorbenzol
0,37 % Trichlorbenzole und höhere Chlorbenzole
Das o/p-Verhältnis betrug somit nur o/p = 0,41.

## Patentansprüche

1. Verfahren zur Herstellung von Dichlorbenzol mit erhöhtem p-Anteil durch Kernchlorierung von Benzol oder Chlorbenzol in Gegenwart von Friedel-Crafts-Katalysatoren und in Gegenwart von Co-Katalysatoren in flüssiger Phase, dadurch gekennzeichnet, daß man als Co-Katalysator eines oder mehrere substituierte Phenothiazine der Formel 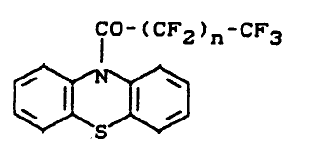 einsetzt, in der
n den Wert Null, Eins oder Zwei annimmt.

2. Verfahren nach Anspruch 1, dadurch gekennzeichnet, daß man Co-Katalysator N-Trifluoracetyl-phenothiazin einsetzt.

3. Verfahren nach Anspruch 1, dadurch gekennzeichnet, daß man als Friedel-Crafts-Katalysator Eisen oder Eisen-(III)-chlorid einsetzt.

4. Verfahren nach Anspruch 1, dadurch gekennzeichnet, daß der Friedel-Crafts-Katalysator in einer Menge von 0,001-1 Gew.-%, bevorzugt in einer Menge von 0,01-0,1 Gew.-%, bezogen auf die Menge des Benzols oder Chlorbenzols, eingesetzt wird.

5. Verfahren nach Anspruch 1, dadurch gekennzeichnet, daß der Co-Katalysator in einer Menge von 0,001-2 Gew.-%, bevorzugt in einer Menge von 0,01-0,2 Gew.-%, bezogen auf die Menge an Benzol oder Chlorbenzol, eingesetzt wird.

6. Verfahren nach Anspruch 1, dadurch gekennzeichnet, daß das Molverhältnis von Friedel-Crafts-Katalysator zu Co-Katalysator wie 10:1 bis 1:10, bevorzugt 2:1 bis 1:2, besonders bevorzugt etwa 1:1 gewählt wird.

7. Verfahren nach Anspruch 1, dadurch gekennzeichnet, daß man bei einer Temperatur zwischen dem Erstarrungspunkt und dem Siedepunkt des Reaktionsgemisches, bevorzugt bei 10-80°C, besonders bevorzugt bei 40-70°C, arbeitet.

## Claims

1. Process for preparing dichlorobenzene having an increased p content by ring chlorination of benzene or chlorobenzene in the presence of Friedel-Crafts catalysts and in the presence of co-catalysts in liquid phase, characterized in that one or more substituted phenothiazines of the formula 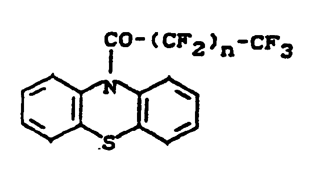 in which
n is zero, one or two,
are used as co-catalyst.

2. Process according to Claim 1, characterized in that the co-catalyst used is N-trifluoroacetyl-phenothiazine.

3. Process according to Claim 1, characterized in that the Friedel-Crafts catalyst used is iron or iron(III) chloride.

4. Process according to Claim 1, characterized in that the Friedel-Crafts catalyst is used in an amount of 0.001-1% by weight, preferably in an amount-of 0.01 to 0.1% by weight, relative to the amount of benzene or chlorobenzene.

5. Process according to Claim 1, characterized in that the co-catalyst is used in an amount of 0.001 to 2% by weight, preferably in an amount of 0.01 to 0.2% by weight, relative to the amount of benzene or chlorobenzene.

6. Process according to Claim 1, characterized in that the molar ratio of Friedel-Crafts catalyst to co-catalyst is selected to be 10:1 to 1:10, preferably 2:1 to 1:2, particularly preferably about 1:1.

7. Process according to Claim 1, characterized in that the reaction is carried out at a temperature between the solidification point and the boiling point of the reaction mixture, preferably at 10-80°C, particularly preferably at 40-70°C.

## Revendications

1. Procédé de préparation de dichlorobenzène avec une teneur en isomère p élevée par chloration sur le noyau de benzène ou de chlorobenzène en présence de catalyseurs de Friedel et Crafts et en présence de co-catalyseurs en phase liquide, caractérisé en ce qu'on utilise en tant que co-catalyseur une ou plusieurs phénothiazines substituées de formule 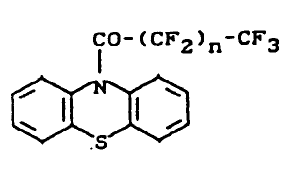 dans laquelle
n vaut 0, 1 ou 2.

2. Procédé selon la revendication 1, caractérisé en ce qu'on utilise comme co-catalyseur la N-trifluoroacétylphénothiazine.

3. Procédé selon la revendication 1, caractérisé en ce qu'on utilise comme catalyseur de Friedel et Crafts du fer ou du chlorure de fer (III).

4. Procédé selon la revendication 1, caractérisé en ce qu'on utilise le catalyseur de Friedel et Crafts en une quantité de 0,001 à 1% en poids, de préférence en une quantité de 0,01 à 0,1% en poids par rapport à la quantité de benzène ou de chlorobenzène.

5. Procédé selon la revendication 1, caractérisé en ce qu'on utilise le co-catalyseur en une quantité de 0,001 à 2 % en poids, de préférence en une quantité de 0,01 à 0,2 % en poids par rapport à la quantité de benzène ou de chlorobenzène.

6. Procédé selon la revendication 1, caractérisé en ce qu'on choisit le rapport molaire du catalyseur de Friedel et Crafts au co-catalyseur entre 10:1 et 1:10, de préférence 2:1 et 1:2, mieux environ 1:1.

7. Procédé selon la revendication 1, caractérisé en ce qu'on travaille à une température comprise entre le point de solidification et le point d'ébullition du mélange réactionnel, de préférence entre 10 et 80°C, mieux entre 40 et 70°C.
